# EUROPÄISCHE PATENTSCHRIFT

(11) **EP 2 337 593 B1**
(45) Veröffentlichungstag und Bekanntmachung des Hinweises auf die Patenterteilung: **18.05.2016**
(21) Anmeldenummer: 09752721.2
(22) Anmeldetag: 08.10.2009
(51) Int. Cl.: A61M 1/34, A61M 1/36, A61M 1/16

(54) **VERFAHREN UND VORRICHTUNG ZUR ÜBERPRÜFUNG DER ZUFUHR VON SUBSTITUTIONSFLÜSSIGKEIT STROMAUF ODER STROMAB EINES DIALYSATORS ODER FILTERS**
METHOD AND DEVICE FOR MONITORING THE INTRODUCTION OF SUBSTITUTION FLUIDS UPSTREAM OR DOWNSTREAM OF A DIALYZER OR FILTER
PROCÉDÉ ET DISPOSITIF DE VÉRIFICATION DE L'APPORT DE LIQUIDE DE SUBSTITUTION EN AMONT OU EN AVAL D'UN DIALYSEUR OU FILTRE

(30) Priorität: 14.10.2008 DE 102008051541
(43) Veröffentlichungstag der Anmeldung: 29.06.2011
(73) Patentinhaber: Fresenius Medical Care Deutschland GmbH, 61352 Bad Homburg v.d.H. (DE)
(72) Erfinder: BEDEN, Josef, 55252 Mainz-Kastel (DE); BADO, Itka, 60385 Frankfurt (DE); VERCH, Georg, 65191 Wiesbaden (DE)
(74) Vertreter: Oppermann, Frank
(86) Internationale Anmeldenummer: PCT/EP2009/007214
(87) Internationale Veröffentlichungsnummer: WO 2010/043331

(56) Entgegenhaltungen:
- EP-A1- 0 189 561
- EP-A1- 1 348 458
- EP-A1- 1 595 560

## Beschreibung

Die Erfindung betrifft ein Verfahren zur Überprüfung der Zufuhr von Substitutionsflüssigkeit für eine extrakorporale Blutbehandlungsvorrichtung mit einem extrakorporalen Blutkreislauf, der eine arterielle Blutleitung, die von einem arteriellen Patientenanschluss zu einer ersten Kammer eines durch eine Membran in die erste Kammer und eine zweite Kammer unterteilten Dialysators oder Filters führt, und eine venöse Blutleitung aufweist, die von der ersten Kammer des Dialysators oder Filters zu einem venösen Patientenanschluss führt. Darüber hinaus betrifft die Erfindung eine Vorrichtung zur Überprüfung der Zufuhr von Substitutionsflüssigkeit für eine extrakorporale Blutbehandlungsvorrichtung mit einem extrakorporalen Blutkreislauf sowie eine extrakorporale Blutbehandlungsvorrichtung mit einer Vorrichtung zur Überprüfung der Zufuhr von Substitutionsflüssigkeit.

Zur Entfernung von harnpflichtigen Substanzen und zum Flüssigkeitsentzug werden beim chronischen Nierenversagen verschiedene Verfahren zur extrakorporalen Blutbehandlung bzw. -reinigung eingesetzt. Bei der Hämodialyse wird das Blut des Patienten außerhalb des Körpers in einem Dialysator gereinigt. Der Dialysator weist eine Blutkammer und eine Dialysierflüssigkeitskammer auf, die von eine semipermeablen Membran getrennt sind. Bei der Behandlung strömt das Blut des Patienten durch die Blutkammer. Um das Blut effektiv von harnpflichtigen Substanzen zu reinigen, wird die Dialysierflüssigkeitskammer kontinuierlich von frischer Dialysierflüssigkeit durchströmt.

Während bei der Hämodialyse (HD) der Transport der kleinen molekularen Substanzen durch die Membran im Wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe durch einen hohen Flüssigkeitsstrom (Konvektion) durch die Membran des Dialysators effektiv entfernt. Bei der Hämofiltration fungiert der Dialysator als Filter. Eine Kombination aus beiden Verfahren ist die Hämodiafiltration (HDF).

Bei der Hämo(dia)filtration wird ein Teil des durch die Membran abgezogenen Serums durch eine sterile Substitutionsflüssigkeit ersetzt, die entweder stromauf des Dialysators oder stromab des Dialysators dem extrakorporalen Blutkreislauf zugeführt wird. Die Zufuhr der Substitutionsflüssigkeit stromauf des Dialysators wird auch als Predilution und die Zufuhr stromab des Dialysators als Postdilution bezeichnet.

Bei den bekannten Hämo(dia)filtrationsvorrichtungen wird die Substitutionsflüssigkeit dem extrakorporalen Blutkreislauf von dem Flüssigkeitssystem der Maschine über eine Substitutionsflüssigkeitsleitung zugeführt. Bei der Predilution führt die Substitutionsflüssigkeitsleitung zu einer Anschlussstelle an der arteriellen Blutleitung stromauf des Dialysators oder Filters, während bei der Postdilution die Substitutionsflüssigkeitsleitung zu einer Anschlussstelle an der venösen Blutleitung stromab des Dialysators führt. Die Substitutionsflüssigkeitsleitung weist im Allgemeinen einen Konnektor auf, mit dem sie entweder an die venöse oder arterielle Blutleitung angeschlossen werden kann. Zum Unterbrechen der Flüssigkeitszufuhr ist an der Substitutionsflüssigkeitsleitung eine Klemme oder dgl. vorgesehen. Ein derartiges Hämo(dia)filtrationsgerät ist beispielsweise aus der EP-A-0 189 561 bekannt.

Die Überwachung der Blutbehandlung setzt die Kenntnis voraus, ob die Substitutionsflüssigkeit stromauf oder stromab des Dialysators oder Filters dem extrakorporalen Blutkreislauf zugeführt wird. Die EP-A-1 348 458 beschreibt ein Verfahren und eine Vorrichtung zur Überwachung der Zufuhr von Substitutionsflüssigkeit für eine extrakorporale Blutbehandlungsvorrichtung. Für die Detektion der Zufuhr von Substitutionsflüssigkeit stromauf oder stromab des Dialysators oder Filters wird die Laufzeit der Druckwellen einer in der Substitutionsflüssigkeitsleitung angeordneten Substituatpumpe gemessen. Die Zufuhr von Substitutionsflüssigkeit stromauf oder stromab des Dialysators oder Filters wird auf der Grundlage der Laufzeitmessung erkannt. Das bekannte Verfahren setzt den Einsatz einer Druckwellen erzeugenden Substituatpumpe voraus.

Die aus der EP-A-1 595 560 bekannte Blutbehandlungsvorrichtung sieht für die Detektion einer Pre- oder Postdilution vor, den Druck im Blutkreislauf stromab des Dialysators oder Filters zu messen, wobei auf eine Pre- oder Postdilution auf der Grundlage der Veränderung des Drucks nach dem Abschalten und/oder Einschalten der für die Förderung der Substitutionsflüssigkeit vorgesehenen Substituatpumpe erkannt wird.

In der Praxis wird die Pre- oder Postdilution durch den Anwender dadurch vorgegeben, dass die Substitutionszuführleitung entweder stromauf des Dialysators oder Filters an die arterielle Blutleitung oder stromab des Dialysators oder Filters an die venöse Blutleitung angeschlossen wird. Weiterhin gibt der Anwender die für die jeweilige Blutbehandlung erforderlichen Behandlungsparameter vor. Dabei besteht die Gefahr, dass nach Einlegen des zur einmaligen Verwendung bestimmten Schlauchsystems, das die arterielle und venöse Blutleitung sowie die Substitutionszuführleitung umfasst, die Substitutionszuführleitung für eine Blutbehandlung mit einer Predilution nicht an der arteriellen Zugabestelle, sondern an der venösen Zugabestelle angeschlossen wird. Umgekehrt besteht die Gefahr, dass für eine Postdilution, die Substitutionsleitung nicht an der venösen, sondern arteriellen Zugabestelle angeschlossen wird. Wenn der falsche Anschluss der Substitutionsleitung nicht bemerkt wird, kann es zu größeren Komplikationen kommen. Beispielsweise kann es bei einer Postdilution mit zu hohen Substitutionsflüssen zu einer starken Hämokonzentration und Koagulation im Dialysator oder Filter kommen.

Der Erfindung liegt die Aufgabe zu Grunde, ein Verfahren anzugeben, das die Überprüfung der Zufuhr von Substitutionsflüssigkeit stromauf oder stromab des Dialysators oder Filters einer extrakorporalen Blutbehandlungsvorrichtung auf einfache Weise mit hoher Sicherheit erlaubt. Darüber hinaus ist eine Aufgabe der Erfindung, eine Vorrichtung zur Überprüfung der Zufuhr von Substitutionsflüssigkeit stromauf oder stromab des Dialysators oder Filters sowie eine extrakorporale Blutbehandlungsvorrichtung zu schaffen, die eine Überprüfung der Zufuhr von Substitutionsflüssigkeit stromauf oder stromab des Dialysators oder Filters erlaubt.

Die Lösung dieser Aufgaben erfolgt erfindungsgemäß mit den Merkmalen der Patentansprüche 1, 7 und 11. Vorteilhafte Ausführungsformen der Erfindung sind Gegenstand der Unteransprüche.

Das erfindungsgemäße Verfahren und die erfindungsgemäße Vorrichtung zur Überprüfung der Zufuhr von Substitutionsflüssigkeit beruhen auf der Überwachung des Flüssigkeitsstands in dem Blasenfänger, der in der venösen Blutleitung des extrakorporalen Blutkreislaufs angeordnet ist. Das erfindungsgemäße Verfahren und die erfindungsgemäße Vorrichtung setzen voraus, dass der extrakorporale Blutkreislauf mit einer Flüssigkeit befüllt wird. Eine Befüllung des extrakorporalen Blutkreislaufs mit einer Flüssigkeit ist insbesondere dann erforderlich, wenn der Blutkreislauf gespült werden soll. Da die arterielle und venöse Blutleitung zusammen mit dem Dialysator oder Filter nach dem Einlegen des Blutschlauchssystems in die Blutbehandlungsvorrichtung im Allgemeinen vor Beginn der extrakorporalen Blutbehandlung gespült werden, stellt die Befüllung des extrakorporalen Blutkreislaufs mit der Spülflüssigkeit keinen zusätzlichen Verfahrensschritt dar, der nur für die Überprüfung der Zufuhr von Substitutionsflüssigkeit erforderlich wäre.

Das erfindungsgemäße Verfahren und die erfindungsgemäße Vorrichtung beruhen weiterhin darauf, dass Flüssigkeit dem extrakorporalen Blutkreislauf über die Substitutionszuführleitung zugeführt wird. Wenn der extrakorporale Blutkreislauf über die Substitutionszuführleitung stromauf des Dialysators oder Filters mit Flüssigkeit befüllt wird, kann die Flüssigkeit über den Dialysator oder Filter bis zu dem Blasenfänger strömen, so dass dieser Teil des Blutkreislaufs vollständig mit Flüssigkeit gefüllt ist. Wenn nun die Blutpumpe zur Förderung der Flüssigkeit in dem extrakorporalen Blutkreislauf in Betrieb gesetzt wird, fällt der Flüssigkeitpegel, der sich zuvor in dem Blasenfänger eingestellt hat, nicht ab. Wenn aber die Flüssigkeit über die Substitutionszuführleitung stromab des Dialysators oder Filters dem extrakorporalen Blutkreislauf zugeführt wird, ist ein großer Teil des Schlauchsystems nicht mit Flüssigkeit gefüllt. Die Blutpumpe fördert dann ein entsprechendes Luftvolumen in den Blasenfänger, so dass der Flüssigkeitsspiegel abfällt. Folglich kann auf eine Predilution dann geschlossen werden, wenn der Füllstand nicht unter einen vorbestimmten Pegel absinkt. Wenn der Füllstand hingegen unter einen vorbestimmten Pegel absinkt, kann auf eine Postdilution geschlossen werden.

Wenn in diesem Zusammenhang von einem Blasenfänger die Rede ist, wird unter einem Blasenfänger jede Einrichtung verstanden, die dazu dient, das im extrakorporalen Blutkreislauf strömende Blut von Blasen zu befreien, bevor das Blut dem Patienten zugeführt wird.

Die Überwachung des Füllstands in dem Blasenfänger kann mit den bekannten Überwachungseinrichtungen erfolgen. Diese sind bei den bekannten Blutbehandlungsvorrichtungen ohnehin vorhanden.

Um sicher zwischen einer Pre- und einer Postdilution unterscheiden zu können, wird vorzugsweise ein vorbestimmter Grenzwert vorgegeben, unter den der Füllstand unter den vorbestimmten Flüssigkeitspegel absinken muss, um auf eine Postdilution zu schließen. Dadurch wird vermieden, dass kleinere Schwankungen des Füllstands zu falschen Schlüssen führen.

Eine bevorzugte Ausführungsform der Erfindung sieht vor, dass der Betriebszustand der Prä- oder Postdilution signalisiert wird. Hierzu kann eine optische und/oder akustische Signaleinheit vorgesehen sein.

Eine weitere bevorzugte Ausführungsform der Erfindung sieht vor, dass ein optischer und/oder ein akustischer Alarm gegeben wird, wenn nicht der Betriebszustand der Pre- oder Postdilution erkannt wird, der vom Anwender vorgegeben worden ist. Der Betriebszustand der Pre- oder Postdilution kann vom Anwender beispielsweise dadurch vorgegeben werden, dass auf einer Bedieneinheit der entsprechende Betriebszustand ausgewählt wird. Hierzu können an der Bedieneinheit Mittel vorgesehen sein, mit denen eine Pre- oder Postdilution eingegeben werden kann, beispielsweise Taster oder Schalter. Es ist aber auch möglich, durch die Vorgabe bestimmter Parameter, die für eine Blutbehandlung mit einer Pre- bzw. Postdilution erforderlich sind, den Betriebszustand der Pre- oder Postdilution vorzugeben.

Das erfindungsgemäße Verfahren und die erfindungsgemäße Vorrichtung können bei allen Behandlungsverfahren Anwendung finden, bei denen das Blut des Patienten in einem extrakorporalen Blutkreislauf strömt.

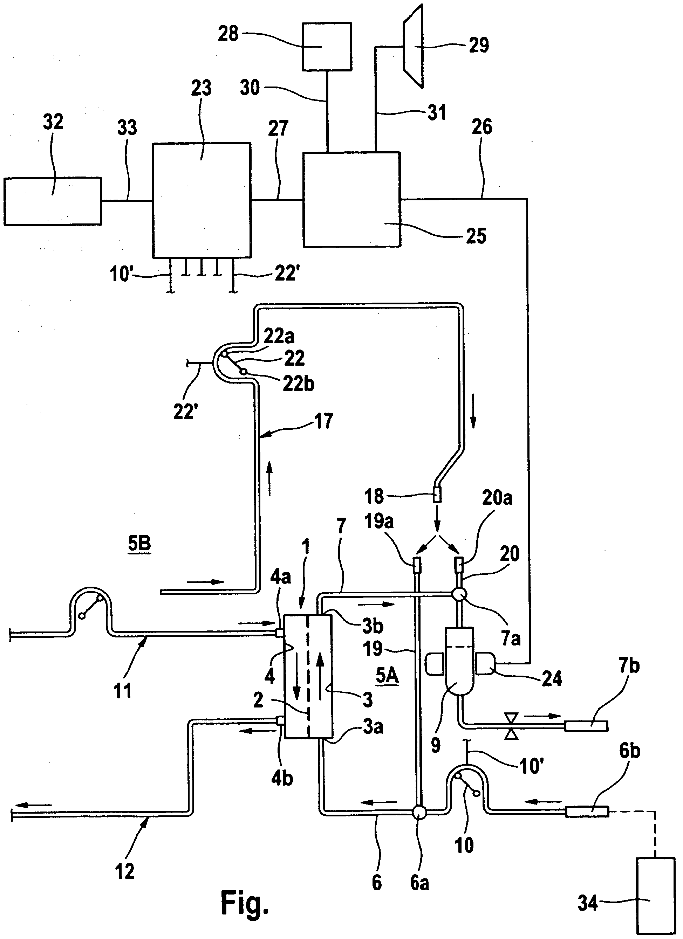

Im Folgenden wird ein Ausführungsbeispiel der Erfindung unter Bezugnahme auf die einzige Zeichnung näher erläutert, die eine extrakorporale Blutbehandlungsvorrichtung zusammen mit einer Vorrichtung zur Überprüfung der Zufuhr von Substitutionsflüssigkeit in stark vereinfachter schematischer Darstellung zeigt.

Die Figur zeigt eine vereinfachte schematische Darstellung der wesentlichen Komponenten einer Hämo(dia)filtrationsvorrichtung zusammen mit einer Einrichtung zur Überprüfung der Zufuhr von Substitutionsflüssigkeit in den extrakorporalen Blutkreislauf stromauf oder stromab des Dialysators oder Filters. Wenn nachfolgend von einem Dialysator die Rede ist, wird darunter auch ein Filter verstanden.

Die Hämo(dia)filtrationsvorrichtung weist einen Dialysator 1 auf, der durch eine semipermeable Membran 2 in eine von Blut durchflossene erste Kammer 3 und eine von Dialysierflüssigkeit durchflossene zweite Kammer 4 getrennt ist. Die erste Kammer 3 ist in einen extrakorporalen Blutkreislauf 5A geschaltet, während die zweite Kammer 4 in das Flüssigkeitssystem 5B der Hämo(dia)filtrationsvorrichtung geschaltet ist.

Der extrakorporale Blutkreislauf 5A umfasst eine arterielle Blutleitung 6, die von einem arteriellen Patientenanschluss 6b zu dem Einlass 3a der Blutkammer 3 führt, und eine venöse Blutleitung 7, die von dem Auslass 3b der Blutkammer 3 des Dialysators 1 zu einem venösen Patientenanschluss 7b führt. Zur Eliminierung von Luftblasen ist in die venöse Blutleitung 7 ein venöser Blasenfänger 9 (Tropfkammer) geschaltet. Das Blut des Patienten wird durch die Blutkammer des Dialysators mittels einer arteriellen Blutpumpe 10, insbesondere Rollenpumpe gefördert, die an der arteriellen Blutleitung 6 angeordnet ist.

Das Flüssigkeitssystem 5B umfasst eine Dialysierflüssigkeitszuführleitung 11, die zu dem Einlass 4a der Dialysierflüssigkeitskammer 4 führt, und eine Dialysierflüssigkeitsabführleitung 12, die von dem Auslass 4b der Dialysierflüssigkeitskammer 4 des Dialysators 1 abgeht. Über die Dialysierflüssigkeitszuführleitung 11 strömt frische Dialysierflüssigkeit aus einer nicht dargestellten Dialysierflüssigkeitsquelle in die Dialysierflüssigkeitskammer, während die verbrauchte Dialysierflüssigkeit aus der Dialysierflüssigkeitskammer über die Dialysierflüssigkeitsabführleitung 12 zu einem nicht dargestellten Abfluss abgeführt wird. Die in den Hämo(dia)filtrationsvorrichtungen im Allgemeinen vorgesehene Bilanzierungseinrichtung zur Bilanzierung frischer gegen verbrauchte Dialysierflüssigkeit ist der besseren Übersichtlichkeit halber nicht dargestellt. Auch sind zusätzliche Einrichtungen zum Reinigen und Spülen des Systems nicht dargestellt.

Das Flüssigkeitssystem 5B umfasst weiterhin eine Substitutionsflüssigkeitszuführleitung 17, die zum extrakorporalen Blutkreislauf 5A führt. Während der Dialysebehandlung kann Substitutionsflüssigkeit aus dem Flüssigkeitssystem 5B über die Substitutionsflüssigkeitszuführleitung 17 dem extrakorporalen Blutkreislauf 5A zugeführt werden. Mit dem Konnektor 18 kann die Substitutionsflüssigkeitsleitung 17 an eine zu der arteriellen Blutleitung 6 führende Anschlussleitung 19 bzw. eine zu der venösen Blutleitung 7 führende Anschlussleitung 20 angeschlossen werden. Die Anschlussleitungen 19, 20 verfügen dafür über entsprechende Anschlussstücke 19a, 20a.

Die Substitutionsflüssigkeit wird mittels einer Okklusionspumpe, insbesondere Rollenpumpe 22 gefördert, in die die Substitutionsflüssigkeitsleitung 17 eingelegt ist.

Die Blutpumpe 10 und die Substituatpumpe 22 sind über Steuerleitungen 10', 22'mit einer zentralen Steuer- und Regeleinheit 23 verbunden, von der die einzelnen Komponenten unter Berücksichtigung der vorgegebenen Behandlungsparameter angesteuert werden.

Die Vorrichtung zur Überprüfung der Zufuhr von Substitutionsflüssigkeit stromauf oder stromab des Dialysators 3 kann eine selbständige Einheit bilden, aber auch Bestandteil der Blutbehandlungsvorrichtung sein. Da einzelne Komponenten der Vorrichtung zur Erkennung der Pre- oder Postdilution in den bekannten Blutbehandlungsvorrichtungen ohnehin vorhanden sind, kann die Vorrichtung zur Erkennung einer Pre- oder Postdilution einfach in die bekannten Blutbehandlungsvorrichtungen integriert werden.

Die Vorrichtung zur Überprüfung der Zufuhr von Substitutionsflüssigkeit weist eine Überwachungseinrichtung 24 zum Überwachen des Füllstandes in der Tropfkammer (Blasenfänger) 9 und eine Auswerteinrichtung 25 auf, die über eine Datenleitung 26 mit der Überwachungseinrichtung 24 verbunden ist. Die Auswerteinrichtung 25 ist wiederum mit einer Datenleitung 27 mit der zentralen Steuer- und Recheneinheit 23 verbunden.

Darüber hinaus verfügt die Vorrichtung zur Überprüfung der Zufuhr von Substitutionsflüssigkeit über eine Signaleinheit 28 und eine Alarmeinheit 29, die mit der Auswerteinrichtung 25 über Datenleitungen 30, 31 verbunden sind.

Die für die Blutbehandlung erforderlichen Behandlungsparameter werden auf einer Eingabeeinheit 32, beispielsweise einer Tastatur und/oder einer Anordnung von Schaltern und Tastern, eingegeben. Die Eingabeeinheit 32 ist mit der zentralen Steuer- und Regeleinheit 23 über eine Datenleitung 33 verbunden.

Nachfolgend wird die Funktionsweise der Vorrichtung zur Überprüfung der Zufuhr von Substitutionsflüssigkeit im Einzelnen beschrieben.

Zur Durchführung der extrakorporalen Blutbehandlung werden zunächst die erforderlichen Behandlungsparameter auf der Eingabeeinheit 32 eingegeben, die von der zentralen Steuer- und Regeleinheit 23 verarbeitet werden. Der Benutzer kann u.a. angeben, ob die Blutbehandlung mit einer Predilution oder einer Postdilution durchgeführt werden soll. Diese Vorgabe einer Pre- oder Postdilution wird von der Auswerteinrichtung 25 ausgelesen.

Der Benutzer legt nun das Blutschlauchsystem, das die arterielle und venöse Blutleitung 6, 7 und die Substitutionszuführleitung 17 umfasst, in die Hämo(dia)filtrationsvorrichtung ein. In Abhängigkeit von der Vorgabe einer Predilution bzw. einer Postdilution muss der Benutzer die Substitutionsflüssigkeitsleitung 17 entsprechend an die Anschlussleitung 19 oder Anschlussleitung 20 anschließen.

Vor Beginn der Blutbehandlung wird der extrakorporale Blutkreislauf 5A gespült. Hierzu wird ein nur andeutungsweise dargestellter Beutel 34, der mit einer Spülflüssigkeit gefüllt ist, an den arteriellen Patientenanschluss 6b der arteriellen Blutleitung 6 angeschlossen. Daraufhin wird die Blutpumpe 10 in Betrieb gesetzt, die Spülflüssigkeit aus dem Beutel 34 fördert. Die Blutpumpe 10 wird so lange betrieben, wie der Leitungsabschnitt der arteriellen Blutleitung 6, der stromauf der arteriellen Zugabestelle 6a liegt, vollständig mit Spülflüssigkeit gefüllt ist. Danach wird die Blutpumpe angehalten. Die weitere Befüllung des extrakorporalen Blutkreislaufs 5A erfolgt nun durch die Substitutionsflüssigkeit über die Substitutionszuführleitung 17. Hierzu wird die Substituatpumpe 22 in Betrieb gesetzt, die Substitutionsflüssigkeit über die Substitutionszuführleitung 17 fördert. Die Substitutionsflüssigkeit strömt in Abhängigkeit von dem Betriebszustand der Pre- oder Postdilution entweder über die arterielle Zugabestelle 6a oder die venöse Zugabestelle 7a in die arterielle bzw. venöse Blutleitung 6, 7.

Die Substituatpumpe 17 wird solange betrieben, bis sich in der Tropfkammer 9 ein vorbestimmter Flüssigkeitspegel eingestellt hat, der mit der Überwachungseinrichtung 24 überwacht wird. Nach Erreichen des vorgegebenen Flüssigkeitspegels erzeugt die Überwachungseinrichtung 24 ein Signal, das die zentrale Steuer- und Regeleinheit 23 veranlasst, die Substituatpumpe 22 zu stoppen. Nunmehr kann die Blutpumpe 10 erneut in Betrieb gesetzt werden, um den extrakorporalen Blutkreislauf zu spülen. Während des Spülvorgangs wird der Füllstand in der Tropfkammer überwacht.

Wenn der Benutzer den Betriebszustand der Predilution vorgegeben hat, darf der Füllstand in der Tropfkammer 9 nach dem Einschalten der Blutpumpe 10 nicht unter einen definierten Grenzwert absinken, da der extrakorporale Blutkreislauf 5A zuvor über die an der arteriellen Zugabestelle 6a angeschlossene Substitutionszuführleitung 17 vollständig mit Flüssigkeit gefüllt worden ist. Wenn der Benutzer aber die Substitutionszuführleitung 17 nicht mit der arteriellen Blutleitung 6, sondern der venösen Blutleitung 7 verbunden hat, d.h. für den Betriebszustand der Predilution nicht Konnektor 18 an 19a, sondern an 20a angeschlossen hat, ist das Schlauchsystem nicht vollständig mit Flüssigkeit befüllt worden, da die Substitutionsflüssigkeit dann nicht stromauf des Dialysators 3, sondern stromab des Dialysators zugeführt worden ist. In diesem Fall sinkt der Flüssigkeitspegel in der Tropfkammer 9 ab, da nach dem Einschalten der Blutpumpe 10 das Luftvolumen in die Tropfkammer 9 gelangt, das in dem Leitungsabschnitt der arteriellen Blutleitung 6 stromab der arteriellen Zugabestelle 6a und dem Leitungsabschnitt der venösen Blutleitung 7 stromauf der venösen Zugabestelle 7a und dem Dialysator eingeschlossen ist.

Die Überwachungseinrichtung 24 vergleicht den vorbestimmten Füllstand mit einem Referenzwert. Wenn die Differenz zwischen dem vorbestimmten Füllstand und dem Referenzwert größer als ein vorgegebener Grenzwert wird, d.h. der Füllstand um einen vorgegebenen Wert abgesunken ist, erzeugt die Überwachungseinrichtung 24 ein Signal, das die Auswerteinrichtung 25 empfängt. Daraufhin gibt die Alarmeinheit 29 einen akustischen und/oder optischen Alarm, der dem Benutzer signalisiert, dass er das Schlauchsystem falsch angeschlossen hat. Weiterhin kann die Auswerteinrichtung 25 ein Signal erzeugen, das die zentrale Steuer- und Regeleinheit 23 veranlasst, den Betrieb der Dialysevorrichtung zu unterbrechen.

Wenn der Benutzer hingegen den Betriebszustand der Postdilution vorgegeben hat, muss der Flüssigkeitsstand beim Einschalten der Blutpumpe 10 zur Durchführung des Spülvorgangs absinken, da in diesem Fall wegen der nicht vollständigen Befüllung des Schlauchsystems über die an der venösen Zugabestelle 7a angeschlossene Substitutionszuführleitung 17 das in den Schlauchleitungsabschnitten eingeschlossene Luftvolumen in die Tropfkammer 9 gelangt. Die Auswerteinrichtung 25 erzeugt in diesem Fall ein Alarmsignal, wenn der Flüssigkeitsstand nicht um den vorgegebenen Grenzwert absinkt. Daraufhin erzeugt die Alarmeinheit 29 wieder einen Alarm.

Auf der Signaleinheit 28 kann angezeigt werden, ob der Benutzer das Schlauchsystem für eine Predilution oder eine Postdilution angeschlossen hat. Wenn die Überwachungseinrichtung 24 beim Betrieb der Blutpumpe 10 für den Spülvorgang ein Absinken des Flüssigkeitspegels in der Tropfkammer 9 erkennt, wird der Betriebszustand der Postdilution angezeigt, während der Betriebszustand der Predilution angezeigt wird, wenn der Flüssigkeitspegel in der Tropfkammer nicht absinkt.

## Patentansprüche

1. Verfahren zur Überprüfung der Zufuhr von Substitutionsflüssigkeit für eine extrakorporale Blutbehandlungsvorrichtung, wobei die extrakorporale Blutbehandlungsvorrichtung aufweist:
einen extrakorporalen Blutkreislauf, der eine arterielle Blutleitung, die von einem arteriellen Patientenanschluss zu einer ersten Kammer eines durch eine Membran in die erste Kammer und eine zweite Kammer unterteilten Dialysators oder Filters führt, und eine venöse Blutleitung aufweist, die von der ersten Kammer des Dialysators oder Filters zu einem venösen Patientenanschluss führt,
eine Substitutionsflüssigkeitszuführleitung zum Zuführen von Substitutionsflüssigkeit an einer arteriellen Zugabestelle stromauf des Dialysators oder Filters, an der für eine Predilution Substitutionsflüssigkeit dem extrakorporalen Blutkreislauf zugeführt wird, und/oder an einer venösen Zugabestelle stromab des Dialysators oder Filters, an der für eine Postdilution Substitutionsflüssigkeit dem extrakorporalen Blutkreislauf zugeführt wird,
eine Blutpumpe zum Fördern von Blut im extrakorporalen Blutkreislauf, und
einen Blasenfänger, der in der venösen Blutleitung angeordnet ist,
**dadurch gekennzeichnet,**
**dass** der Teil des extrakorporalen Blutkreislaufs mit einer Flüssigkeit befüllt wird, der zwischen dem arteriellen Patientenanschluss und der arteriellen Zugabestelle des extrakorporalen Blutkreislaufs liegt,
**dass** eine Flüssigkeit dem extrakorporalen Blutkreislauf über die entweder an der arteriellen oder der venösen Zugabestelle angeschlossenen Substitutionsflüssigkeitsleitung solange zugeführt wird, bis sich in dem Blasenfänger ein vorbestimmter Flüssigkeitspegel eingestellt hat,
**dass** die Blutpumpe zur Förderung einer Flüssigkeit in dem extrakorporalen Blutkreislauf in Betrieb gesetzt wird, und
**dass** der Flüssigkeitspegel in dem Blasenfänger überwacht wird, wobei auf einen Anschluss der Substitutionsflüssigkeitsleitung an der arteriellen Zugabestelle geschlossen wird, wenn der Flüssigkeitspegel in dem Blasenfänger nicht unter einen vorbestimmten Füllstand absinkt oder auf einen Anschluss der Substitutionsflüssigkeitsleitung an der venösen Zugabestelle geschlossen wird, wenn der Flüssigkeitspegel in dem Blasenfänger unter den vorbestimmten Füllstand absinkt.

2. Verfahren nach Anspruch 1, **dadurch gekennzeichnet, dass** zum Füllen des Teils des extrakorporalen Blutkreislaufs mit einer Flüssigkeit, der zwischen dem arteriellen Patientenanschluss und der arteriellen Zugabestelle liegt, ein mit einer Flüssigkeit befülltes Behältnis an den arteriellen Patientenanschluss angeschlossen und die Blutpumpe zur Förderung der Flüssigkeit in den extrakorporalen Blutkreislauf in Betrieb gesetzt wird.

3. Verfahren nach Anspruch 2, **dadurch gekennzeichnet, dass** der arterielle Patientenanschluss an einen mit einer Spülflüssigkeit befüllten Beutel angeschlossen wird.

4. Verfahren nach einem der Ansprüche 1 bis 3, **dadurch gekennzeichnet, dass** der Betriebszustand der Predilution signalisiert wird, wenn der Flüssigkeitspegel in dem Blasenfänger nicht unter den vorbestimmten Füllstand absinkt oder der Betriebszustand der Postdilution signalisiert wird, wenn der Flüssigkeitspegel in dem Blasenfänger unter den vorbestimmten Füllstand absinkt.

5. Verfahren nach einem der Ansprüche 1 bis 4, **dadurch gekennzeichnet, dass** für die extrakorporale Blutbehandlungsvorrichtung der Betriebszustand der Predilution vorgegeben wird, wobei ein akustischer und/oder optischer Alarm gegeben wird, wenn der Flüssigkeitspegel in dem Blasenfänger unter den vorbestimmten Füllstand absinkt.

6. Verfahren nach einem der Ansprüche 1 bis 4, **dadurch gekennzeichnet, dass** für die extrakorporale Blutbehandlungsvorrichtung der Betriebszustand der Postdilution vorgegeben wird, wobei ein akustischer und/oder optischer Alarm gegeben wird, wenn der Flüssigkeitspegel in dem Blasenfänger nicht unter den vorbestimmten Füllstand absinkt.

7. Vorrichtung zur Überprüfung der Zufuhr von Substitutionsflüssigkeit für eine extrakorporale Blutbehandlungsvorrichtung, wobei die extrakorporale Blutbehandlungsvorrichtung aufweist:
einen extrakorporalen Blutkreislauf, der eine arterielle Blutleitung, die von einem arteriellen Patientenanschluss zu einer ersten Kammer eines durch eine Membran in die erste Kammer und eine zweite Kammer unterteilten Dialysators oder Filters führt, und eine venöse Blutleitung aufweist, die von der ersten Kammer des Dialysators oder Filters zu einem venösen Patientenanschluss führt,
eine Substitutionsflüssigkeitszuführleitung zum Zuführen von Substitututionsflüssigkeit an einer arteriellen Zugabestelle stromauf des Dialysators oder Filters, an der für eine Predilution Substitutionsflüssigkeit dem extrakorporalen Blutkreislauf zugeführt wird, und/oder an einer venösen Zugabestelle stromab des Dialysators oder Filters, an der bei einer Postdilution Substitutionsflüssigkeit dem extrakorporalen Blutkreislauf zugeführt wird,
eine Blutpumpe zum Fördern von Blut im extrakorporalen Blutkreislauf,
einen Blasenfänger, der in der venösen Blutleitung angeordnet ist, und
eine Überwachungseinrichtung zum Überwachen des Füllstandes in dem Blasenfänger,
**dadurch gekennzeichnet,**
**dass** eine Auswerteinrichtung (25) vorgesehen ist, die mit der Überwachungseinrichtung (24) zum Überwachen des Füllstandes in dem Blasenfänger derart zusammenwirkt, dass auf einen Anschluss der Substitutionsflüssigkeitsleitung an der arteriellen Zugabestelle (6a) geschlossen wird, wenn der Flüssigkeitspegel in dem Blasenfänger (9) nicht unter einen vorbestimmten Füllstand absinkt oder auf einen Anschluss der Substitutionsflüssigkeitsleitung an der venösen Zugabestelle (7a) geschlossen wird, wenn der Flüssigkeitspegel in dem Blasenfänger (9) unter den vorbestimmten Füllstand absinkt.

8. Vorrichtung nach Anspruch 7, **dadurch gekennzeichnet, dass** Mittel (32) zum Vorgeben des Betriebszustands der Predilution oder Postdilution für die extrakorporale Blutbehandlungsvorrichtung vorgesehen sind.

9. Vorrichtung nach Anspruch 8, **dadurch gekennzeichnet, dass** eine Signaleinheit (28) vorgesehen ist, die den Betriebszustand der Predilution signalisiert, wenn der Flüssigkeitspegel in dem Blasenfänger nicht unter den vorbestimmten Füllstand absinkt, oder den Betriebszustand der Postdilution signalisiert, wenn der Flüssigkeitspegel in dem Blasenfänger unter den vorbestimmten Füllstand absinkt.

10. Vorrichtung nach Anspruch 8 oder 9, **dadurch gekennzeichnet, dass** eine Alarmeinheit (29) vorgesehen ist, die derart mit den Mitteln (32) zum Vorgeben des Betriebszustands der Predilution oder Postdilution zusammenwirkt, dass bei der Vorgabe der Predilution ein akustischer und/oder optischer Alarm gegeben wird, wenn der Flüssigkeitspegel in dem Blasenfänger unter den vorbestimmten Füllstand absinkt, oder bei der Vorgabe der Postdilution ein akustischer und/oder optischer Alarm gegeben wird, wenn der Flüssigkeitspegel in dem Blasenfänger nicht unter den vorbestimmten Füllstand absinkt.

11. Extrakorporale Blutbehandlungsvorrichtung mit
einem extrakorporalen Blutkreislauf (5A), der eine arterielle Blutleitung (6), die von einem arteriellen Patientenanschluss (6b) zu einer ersten Kammer (3) eines durch eine Membran (2) in die erste Kammer (3) und eine zweite Kammer (4) unterteilten Dialysators (1) oder Filters führt, und eine venöse Blutleitung (7) aufweist, die von der ersten Kammer (3) des Dialysators (1) oder Filters zu einem venösen Patientenanschluss (7b) führt,
einer Substitutionsflüssigkeitszuführleitung (17) zum Zuführen von Substitututionsflüssigkeit an einer arteriellen Zugabestelle (6a) stromauf des Dialysators oder Filters, an der für eine Predilution Substitutionsflüssigkeit dem extrakorporalen Blutkreislauf zugeführt wird, und/oder an einer venösen Zugabestelle (7a) stromab des Dialysators oder Filters, an der bei einer Postdilution Substitutionsflüssigkeit dem extrakorporalen Blutkreislauf zugeführt wird,
einer Blutpumpe (10) zum Fördern von Blut im extrakorporalen Blutkreislauf (5A),
einem Blasenfänger (9), der in der venösen Blutleitung (7) angeordnet ist, und
einer Überwachungseinrichtung zum Überwachen des Füllstandes in dem Blasenfänger,
**dadurch gekennzeichnet, dass** die extrakorporale Blutbehandlungsvorrichtung eine Vorrichtung zur Überprüfung der Zufuhr von Substitutionsflüssigkeit nach einem der Ansprüche 7 bis 10 aufweist.

12. Vorrichtung nach Anspruch 11, **dadurch gekennzeichnet, dass** die arterielle und venöse Blutleitung (6, 7) Teil eines zur einmaligen Verwendung bestimmten Schlauchsystems sind, das die Substitutionsflüssigkeitszuführleitung (17) umfasst.

## Claims

1. Method for checking the supply of substitution fluid for an extracorporeal blood treatment apparatus, the extracorporeal blood treatment apparatus comprising:
an extracorporeal blood circuit having an arterial blood line that leads from an arterial patient connection to a first chamber of a dialyser or filter that is divided into the first chamber and a second chamber by means of a membrane, and a venous blood line that leads from the first chamber of the dialyser or filter to a venous patient connection,
a substitution fluid supply line for supplying substitution fluid at an arterial addition site upstream of the dialyser or filter, at which site substitution fluid is supplied to the extracorporeal blood circuit for predilution, and/or at a venous addition site downstream of the dialyser or filter, at which site substitution fluid is supplied to the extracorporeal blood circuit for post dilution,
a blood pump for conveying blood in the extracorporeal blood circuit, and
a bubble trap arranged in the venous blood line,
**characterised in that**
the part of the extracorporeal blood circuit located between the arterial patient connection and the arterial addition site of the extracorporeal blood circuit is filled with a fluid,
**in that** a fluid is supplied to the extracorporeal blood circuit via the substitution fluid line connected either to the arterial or venous addition site until a preset fluid level has been reached in the bubble trap,
**in that** the blood pump is actuated in order to convey a fluid in the extracorporeal blood circuit, and
**in that** the fluid level in the bubble trap is monitored, a conclusion being drawn on a connection between the substitution fluid line and the arterial addition site if the fluid level in the bubble trap does not fall below a preset level or a conclusion being drawn on a connection between the substitution fluid line and the venous addition site if the fluid level in the bubble trap does fall below the preset level.

2. Method according to claim 1, **characterised in that**, in order to fill with fluid the part of the extracorporeal blood circuit located between the arterial patient connection and the arterial addition site, a container filled with a fluid is connected to the arterial patient connection and the blood pump is actuated in order to convey the fluid into the extracorporeal blood circuit.

3. Method according to claim 2, **characterised in that** the arterial patient connection is connected to a bag filled with a rinsing fluid.

4. Method according to any of claims 1 to 3, **characterised in that** the predilution operating state is signalled if the fluid level in the bubble trap does not fall below the preset level or the postdilution operating state is signalled if the fluid level in the bubble trap does fall below the preset level.

5. Method according to any of claims 1 to 4, **characterised in that** the predilution operating state is selected for the extracorporeal blood treatment apparatus, an acoustic and/or visual alarm being emitted if the fluid level in the bubble trap falls below the preset level.

6. Method according to any of claims 1 to 4, **characterised in that** the postdilution operating state is selected for the extracorporeal blood treatment apparatus, an acoustic and/or visual alarm being emitted if the fluid level in the bubble trap does not fall below the preset level.

7. Apparatus for checking the supply of substitution fluid for an extracorporeal blood treatment apparatus, the extracorporeal blood treatment apparatus comprising:
an extracorporeal blood circuit having an arterial blood line that leads from an arterial patient connection to a first chamber of a dialyser or filter that is divided into the first chamber and a second chamber by means of a membrane, and a venous blood line that leads from the first chamber of the dialyser or filter to a venous patient connection,
a substitution fluid supply line for supplying substitution fluid at an arterial addition site upstream of the dialyser or filter, at which site substitution fluid is supplied to the extracorporeal blood circuit for predilution, and/or at a venous addition site downstream of the dialyser or filter, at which site substitution fluid is supplied to the extracorporeal blood circuit for postdilution,
a blood pump for conveying blood in the extracorporeal blood circuit,
a bubble trap arranged in the venous blood line, and
a monitoring device for monitoring the level in the bubble trap,
**characterised in that**
an evaluation device (25) is provided that interacts with the monitoring device (24) in order to monitor the level in the bubble trap such that a conclusion is drawn on a connection between the substitution fluid line and the arterial addition site (6a) if the fluid level in the bubble trap (9) does not fall below a preset level or a conclusion is drawn on a connection between the substitution fluid line and the venous addition site (7a) if the fluid level in the bubble trap (9) does fall below the preset level.

8. Apparatus according to claim 7, **characterised in that** means (32) for preselecting the predilution or postdilution operating state for the extracorporeal blood treatment apparatus are provided.

9. Apparatus according to claim 8, **characterised in that** a signal unit (28) is provided that signals the predilution operating state if the fluid level in the bubble trap does not fall below the preset level or that signals the postdilution operating state if the fluid level in the bubble trap does fall below the preset level.

10. Apparatus according to either claim 8 or claim 9, **characterised in that** an alarm unit (29) is provided that interacts with the means (32) for preselecting the predilution or postdilution operating state such that, when predilution is selected, an acoustic and/or visual alarm is emitted if the fluid level in the bubble trap falls below the preset level or, when postdilution is selected, an acoustic and/or visual alarm is emitted if the fluid level in the bubble trap does not fall below the preset level.

11. Extracorporeal blood treatment apparatus comprising
an extracorporeal blood circuit (5A) that has an arterial blood line (6) that leads from an arterial patient connection (6b) to a first chamber (3) of a dialyser (1) or filter that is divided into the first chamber (3) and a second chamber (4) by means of a membrane (2), and a venous blood line (7) that leads from the first chamber (3) of the dialyser (1) or filter to a venous patient connection (7b),
a substitution fluid supply line (17) for supplying substitution fluid at an arterial addition site (6a) upstream of the dialyser or filter, at which site substitution fluid is supplied to the extracorporeal blood circuit for predilution, and/or at a venous addition site (7a) downstream of the dialyser or filter, at which site substitution fluid is supplied to the extracorporeal blood circuit for postdilution,
a blood pump (10) for conveying blood in the extracorporeal blood circuit (5A),
a bubble trap (9) arranged in the venous blood line (7), and
a monitoring device for monitoring the level in the bubble trap,
**characterised in that** the extracorporeal blood treatment apparatus comprises an apparatus for checking the supply of substitution fluid according to any of claims 7 to 10.

12. Apparatus according to claim 11, **characterised in that** the arterial and venous blood lines (6, 7) are each part of a tubing system that is intended for single use and comprises the substitution fluid supply line (17).

## Revendications

1. Procédé de vérification de l'alimentation d'un liquide de substitution pour un dispositif de traitement de sang extracorporel, le dispositif de traitement de sang extracorporel comprenant :
un circuit de circulation sanguine extracorporelle, qui comprend une conduite de sang artériel qui conduit d'un raccord de patient artériel vers une première chambre d'un dialyseur ou d'un filtre divisé par une membrane en une première chambre et une deuxième chambre, et une conduite de sang veineux qui conduit de la première chambre du dialyseur ou du filtre vers un raccord de patient veineux,
une conduite d'alimentation en liquide de substitution pour l'introduction d'un liquide de substitution au niveau d'un emplacement d'alimentation artérielle en amont du dialyseur ou du filtre, au niveau duquel, pour une pré-dilution, un liquide de substitution est ajouté à la circulation sanguine extracorporelle, et/ou au niveau d'un emplacement d'alimentation veineuse en aval du dialyseur ou du filtre, au niveau duquel, pour une post-dilution, un liquide de substitution est ajouté au circuit de circulation sanguine extracorporelle,
une pompe à sang pour le transport du sang dans la circulation extracorporelle, et
un piège à bulles disposé dans la conduite de sang veineux,
**caractérisé en ce que**
la partie de la circulation sanguine extracorporelle qui se trouve entre le raccord de patient artériel et l'emplacement d'alimentation artériel est remplie d'un liquide,
un liquide est ajouté à la circulation extracorporelle par l'intermédiaire soit de l'emplacement d'alimentation artériel soit de l'emplacement d'alimentation veineux jusqu'à ce que, dans le piège à bulles, un niveau de liquide prédéterminé apparaisse,
la pompe à sang est mise en marche pour le transport d'un liquide dans la circulation sanguine extracorporelle, et
le niveau de liquide dans le piège à bulles est surveillé, la conduite de liquide de substitution étant considérée comme raccordée à l'emplacement d'alimentation artériel lorsque le niveau de liquide dans le piège à bulles ne diminue pas en dessous d'un niveau de remplissage prédéterminé ou bien la conduite de liquide de substitution étant considérée comme raccordée à l'emplacement d'alimentation veineux lorsque le niveau de liquide dans le piège à bulles diminue en dessous du niveau de remplissage prédéterminé.

2. Procédé selon la revendication 1, **caractérisé en ce que**, pour le remplissage de la partie de la circulation sanguine extracorporelle avec un liquide, qui se trouve entre le raccord de patient artériel et l'emplacement d'alimentation artériel, un récipient rempli d'un liquide est raccordé au raccord de patient artériel et la pompe à sang est mise en marche pour le transport du liquide dans la circulation sanguine extracorporelle.

3. Procédé selon la revendication 2, **caractérisé en ce que**, le raccord de patient artériel est raccordé à une poche remplie d'un liquide de rinçage.

4. Procédé selon l'une des revendications 1 à 3, **caractérisé en ce que** le mode de fonctionnement de pré-dilution est signalé lorsque le niveau de liquide dans le piège à bulles ne diminue pas en dessous du niveau de remplissage prédéterminé ou le mode de fonctionnement de post-dilution est signalé lorsque le niveau de liquide dans le piège à bulles diminue en dessous du niveau de remplissage prédéterminé.

5. Procédé selon l'une des revendications 1 à 4, **caractérisé en ce que**, pour le dispositif de traitement de sang extracorporel, le mode de fonctionnement « pré-dilution » est prédéterminé, une alarme sonore et/ou optique étant déclenchée lorsque le niveau de liquide dans le piège à bulles diminue en dessous du niveau de remplissage prédéterminé.

6. Procédé selon l'une des revendications 1 à 4, **caractérisé en ce que**, pour le dispositif de traitement de sang extracorporel, le mode de fonctionnement « post-dilution » est prédéterminé, une alarme sonore et/ou optique étant déclenchée lorsque le niveau de liquide dans le piège à bulles ne diminue pas en dessous du niveau de remplissage prédéterminé.

7. Dispositif de vérification de l'alimentation en liquide de substitution pour un dispositif de traitement de sang extracorporel, le dispositif de traitement de sang extracorporel comprenant :
un circuit de circulation sanguine extracorporelle qui comprend une conduite de sang artériel, qui conduit vers une première chambre d'un dialyseur ou d'un filtre divisé par une membrane en une première chambre et une deuxième chambre, et une conduite de sang veineux, qui conduit de la première chambre du dialyseur ou du filtre vers un raccord de patient veineux,
une conduite d'alimentation en liquide de substitution pour l'introduction d'un liquide de substitution au niveau d'un emplacement d'alimentation en amont du dialyseur ou du filtre, au niveau duquel, pour une pré-dilution, un liquide de substitution est ajouté à la circulation sanguine extracorporelle et/ou, au niveau d'un emplacement d'alimentation veineux en aval du dialyseur ou du filtre, au niveau duquel, lors d'une post-dilution, un liquide de substitution est ajouté au circuit de circulation sanguine extracorporelle,
une pompe à sang pour le transport du sang dans la circulation extracorporelle,
un piège à bulles disposé dans la conduite de sang veineux et
un dispositif de surveillance pour la surveillance du niveau de remplissage dans le piège à bulles,
**caractérisé en ce que**
un dispositif d'analyse (25) est prévu, qui interagit avec le dispositif de surveillance (24) pour la surveillance du niveau de remplissage dans le piège à bulles de façon à ce que la conduite de liquide de substitution soit considérée comme raccordée à l'emplacement d'alimentation artériel (6a) lorsque le niveau de liquide dans le piège à bulles (9) ne diminue pas en dessous d'un niveau de remplissage prédéterminé ou bien de façon à ce que la conduite de liquide de substitution soit considérée comme raccordée à l'emplacement d'alimentation veineux (7a) lorsque le niveau de liquide dans le piège à bulles (9) diminue en dessous du niveau de remplissage prédéterminé.

8. Dispositif selon la revendication 7, **caractérisé en ce que** des moyens (32) sont prévus pour la prédétermination du mode de fonctionnement de pré-dilution ou de post-dilution pour le dispositif de traitement de sang extracorporel.

9. Dispositif selon la revendication 8, **caractérisé en ce qu'**une unité de signalisation (28) est prévue, qui signale le mode de fonctionnement « pré-dilution » lorsque le niveau de liquide dans le piège à bulles ne diminue pas en dessous du niveau de remplissage prédéterminé ou bien signale le mode de fonctionnement « post-dilution » lorsque le niveau de liquide dans le piège à bulles diminue en dessous du niveau de remplissage prédéterminé.

10. Dispositif selon la revendication 8 ou 9, **caractérisé en ce qu'**une unité d'alarme (29) est prévue, qui interagit avec les moyens (32) de prédétermination du mode de fonctionnement de pré-dilution ou de post-dilution de façon à ce que, lors de la prédétermination de la pré-dilution, une alarme sonore et/ou optique soit déclenchée lorsque le niveau de liquide dans le piège à bulles diminue en dessous du niveau de remplissage prédéterminé ou bien, lors de la prédétermination de la post-dilution, une alarme sonore et/ou optique soit déclenchée lorsque le niveau de liquide dans le piège à bulles ne diminue pas en dessous du niveau de remplissage prédéterminé.

11. Dispositif de traitement de sang extracorporel avec
un circuit de circulation sanguine extracorporelle (5A), qui comprend une conduite de sang artériel (6) qui conduit d'un raccord de patient artériel (6b) vers une première chambre (3) d'un dialyseur (1) ou d'un filtre divisé par une membrane (2) en une première chambre (3) et une deuxième chambre (4), et une conduite de sang veineux (7) qui conduit de la première chambre (3) du dialyseur (1) ou du filtre vers un raccord de patient veineux (7b),
une conduite d'alimentation en liquide de substitution (17) pour l'introduction d'un liquide de substitution au niveau d'un emplacement d'alimentation artérielle (6a) en amont du dialyseur ou du filtre, au niveau duquel, pour une pré-dilution, un liquide de substitution est ajouté à la circulation sanguine extracorporelle, et/ou au niveau d'un emplacement d'alimentation veineuse (7a) en aval du dialyseur ou du filtre, au niveau duquel, pour une post-dilution, un liquide de substitution est ajouté à la circulation sanguine extracorporelle,
une pompe à sang (10) pour le transport du sang dans la circulation extracorporelle,
un piège à bulles (9) disposé dans la conduite de sang veineux (7) et
un dispositif de surveillance pour la surveillance du niveau de remplissage dans le piège à bulles,
**caractérisé en ce que** le dispositif de traitement de sang extracorporel comprend un dispositif de vérification de l'alimentation en liquide de substitution selon l'une des revendications 7 à 10.

12. Dispositif selon la revendication 11, **caractérisé en ce que** les conduites de sang artériel et veineux (6, 7) font partie d'un système de tuyaux à usage unique, qui comprend la conduite d'alimentation en liquide de substitution (17).
